# EUROPEAN PATENT SPECIFICATION

(11) **EP 4 313 193 B1**
(45) Date of publication and mention of the grant of the patent: **07.10.2026**
(21) Application number: 22776540.1
(22) Date of filing: 23.03.2022
(51) Int. Cl.: A61M 1/00, A61M 1/14, A61M 1/28

(54) **MEDICAMENT PREPARATION DEVICES, METHODS, AND SYSTEMS**
VORRICHTUNGEN, VERFAHREN UND SYSTEME ZUR HERSTELLUNG VON ARZNEIMITTELN
SYSTÈMES, PROCÉDÉS ET DISPOSITIFS DE PRÉPARATION DE MÉDICAMENT

(30) Priority: 23.03.2021 US 202163164936 P
(43) Date of publication of application: 07.02.2024
(73) Proprietor: NxStage Medical, Inc., Lawrence, MA 01843 (US)
(72) Inventor: FRIEDERICHS, Goetz, Waltham, Massachusetts 02451 (US); YANTZ, Gregory, Waltham, Massachusetts 02451 (US)
(74) Representative: Freischem & Partner Patentanwälte mbB
(86) International application number: PCT/US2022/021477
(87) International publication number: WO 2022/204238

(56) References cited:
- WO-A1-2020/237033
- WO-A2-2022/086922
- US-A1- 2019 262 522
- US-A1- 2019 262 522
- US-A1- 2020 016 317

## Description

### Cross-Reference to Related Applications

This application claims the benefit of U.S. Provisional Application No. 63/164,936 filed March 23, 2021.

### Background

The disclosed subject matter relates generally to devices, methods, systems, improvements, and components for preparing medicaments and making medicament available for use by a consumer, for example, a dialysis cycler.

Peritoneal dialysis is a mature technology that has been in use for many years. It is one of two common forms of dialysis, the other being hemodialysis, which uses an artificial membrane to directly cleanse the blood of a renal patient. Peritoneal dialysis employs the natural membrane of the peritoneum to permit the removal of excess water and toxins from the blood.

In peritoneal dialysis, sterile peritoneal dialysis fluid is infused into a patient's peritoneal cavity using a catheter that has been inserted through the abdominal wall. The fluid remains in the peritoneal cavity for a dwell period. Osmotic exchange with the patient's blood occurs across the peritoneal membrane, removing urea and other toxins and excess water from the blood. Ions that need to be regulated are also exchanged across the membrane. The removal of excess water results in a higher volume of fluid being removed from the patient than is infused. The net excess is called ultrafiltrate, and the process of removal is called ultrafiltration. After the dwell time, the dialysis fluid is removed from the body cavity through the catheter.

It is referred to US 2019/0262522 A1 and WO 2020/237033 A1 as prior art.

### Summary

The invention is given by a method comprising the features of claim 1.

Methods, devices, and systems for preparing medicaments such as, but not limited to, dialysis fluid are disclosed. In embodiments, medicament is prepared at a point of care (POC) automatically using a daily sterile disposable fluid circuit, one or more concentrates to make batches of medicament at the POC. The dialysis fluid may be used at the POC for any type of renal replacement therapy, including at least peritoneal dialysis, hemodialysis, hemofiltration, and hemodiafiltration.

In embodiments, peritoneal dialysis fluid is prepared at a point of use automatically using a daily sterile disposable fluid circuit and one or more long-term concentrate containers that are changed only after multiple days (e.g. weekly). The daily disposable may have concentrate containers that are initially empty and are filled from the long-term concentrate containers once per day at the beginning of a treatment.

Embodiments of medicament preparation, devices, systems, and methods are described herein. The features, in some cases, relate to automated dialysis such as peritoneal dialysis, hemodialysis and others, and in particular to systems, methods, and devices that prepare peritoneal dialysis fluid in a safe and automated way at a point of care. The disclosed features may be applied to any kind of medicament system and are not limited to dialysis fluid.

In embodiments, a system that prepares a medical fluid is configured in such a manner that it outputs the medical fluid to a consuming process (for example, a peritoneal dialysis cycler) wherein the consuming process does not distinguish between the system that prepares the medical fluid and pre-packaged bags of dialysate. This allows embodiments of the presently disclosed system for preparing the medical fluid to be used with any type of a cycler, without any special customization or modification of the cycler.

Objects and advantages of embodiments of the disclosed subject matter will become apparent from the following description when considered in conjunction with the accompanying drawings.

### Brief Description of the Drawings

Embodiments will hereinafter be described in detail below with reference to the accompanying drawings, wherein like reference numerals represent like elements. The accompanying drawings have not necessarily been drawn to scale. Where applicable, some features may not be illustrated to assist in the description of underlying features.
Fig. 1A shows a system for preparing a ready-to-use medicament from concentrated medicament and water according to embodiments of the disclosed subject matter.
Fig. 1B shows another example of a system for preparing a ready-to-use medicament from concentrated medicament and water according to embodiments of the disclosed subject matter.
Fig. 2A shows a flow chart of a method for checking the concentration and/or conductivity of medicament according to embodiments of the disclosed subject matter.
Fig. 2B show a flow chart of a method for preparing a ready-to-use medicament according to embodiments of the disclosed subject matter.
Fig. 3 shows a system for generating purified water for the system and method of Figs. 1A and 1B according to embodiments of the disclosed subject matter.
Figs. 4A, 4B, and 4C show configurations of the systems providing water to a mixing container according to embodiments of the disclosed subject matter.
Figs. 5A and 5B show configurations of systems providing various types of medicament concentrate to the mixing container according to embodiments of the disclosed subject matter.
Figs. 6A and 6B show configurations of the systems mixing the content of the mixing container according to embodiments of the disclosed subject matter.
Fig. 7 shows various configurations of the systems testing conductivity of the content of the mixing container according to embodiments of the disclosed subject matter.
Figs. 8A, 8B, and 8C show configurations of the systems providing the content of the mixing container to a consumer of the content according to embodiments of the disclosed subject matter.
Fig. 9 shows a computer system that may describe the functions and elements of a controller as described herein and in accordance with the embodiments of the disclosed subject matter.

### Detailed Description

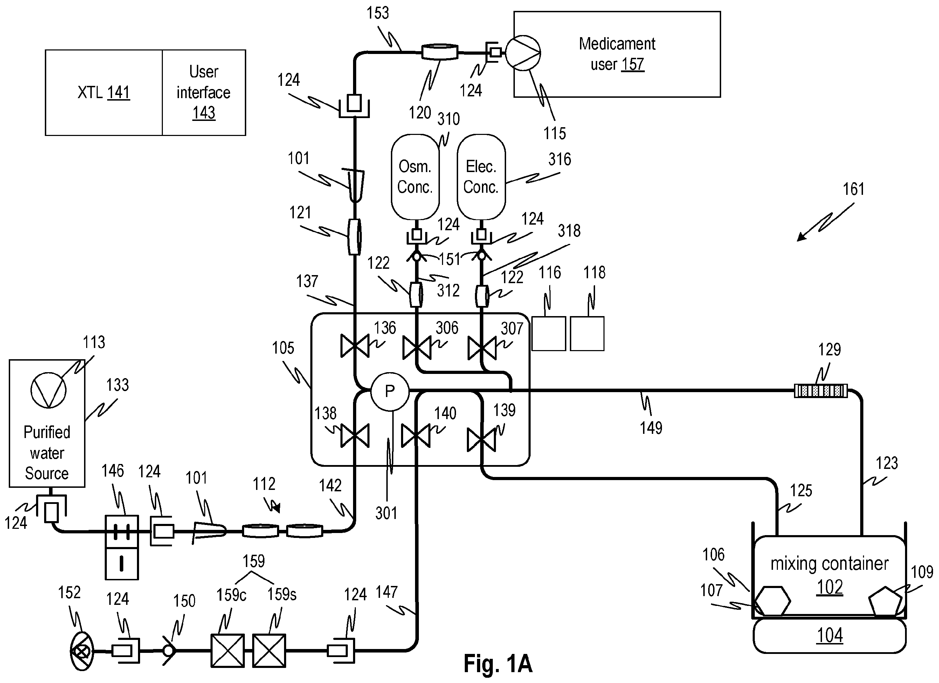

Fig. 1A shows an embodiment of a system that uses water and up to two concentrated medicaments (also referred to as "medicament concentrates" or "concentrates") in containers 310 and 316 to make a therapeutic fluid that can be used for treatment according to embodiments of the disclosed subject matter. In embodiments, the concentrated medicament in container 310 is an osmotic agent. In embodiments, the osmotic agent includes concentrated dextrose solution. In other embodiments, the osmotic agent includes concentrated glucose solution. In embodiments, the concentrated medicament in container 316 is an electrolyte concentrate.

Each of the containers 310 and 316 may be connected to fluid lines 312 and 318 via a connector 124, as shown. However, it is also possible that each or one of the containers are pre-connected to the fluid lines 312 and 318, thus avoiding connectors 124. Osmotic concentrate container 310 is fluidly connected to osmotic fluid line 312. An optional one-way check valve may be provided as shown. Similarly, electrolyte container 316 is fluidly connected to electrolyte fluid line 318 and may include one-way check valve 151. However, these one-way check valves are optional and may be omitted. Way check valve are particularly advantageous when multiple batches of medicament are made without changing concentrate containers, as they prevent contamination from reaching the concentrated medicament containers 310 and 316. Osmotic fluid line 312 is controlled by osmotic valve 306. Electrolyte fluid line 318 is controlled by electrolyte valve 307.

The concentrate lines 312 and 318 may each include an optional filter 122. The filter 122 may be a touch contamination protection filter, such as a 0.2-micron filter.

Still referring to Fig. 1A, a purified water source 133 with a water pump 113 supplies highly purified water through a connector 124 through a water line 142. The water line 142 has a non-reopenable clamp 146, another connector 124, a manual tube clamp 101, and a pair of redundant 0.2-micron sterilizing filters 112, as shown. In embodiments, different types of sterilizing filters may be used, and not limited to 0.2 micron, or to two redundant filters. For example, a single filter may be used, and a testing protocol provided to ensure that the filter does not fail before replacement.

A water inlet clamp 138, batch release clamp 136, and a conductivity sensor clamp 140 are controlled by a controller 141, which may be operatively coupled to a user interface 143, which may include a visual and/or audible output and various devices for receiving user input. The controller 141 controls the pinch clamps and a peristaltic pump 129 to make a batch of diluted concentrate in a mixing container 102 by diluting medicament concentrate (e.g., dialysis fluid concentrate) in the mixing container 102. The mixing container 102 is supplied empty and permanently connected to a fluid circuit that includes fluid lines 149, 123, and 125.

A pressure sensor 301 is provided in the flow path as shown and outputs a signal representative of the pressure in the fluid lines that are fluidly connected to the pressure sensor. This pressure signal may be provided to controller 141.

The mixing container 102 may be a part of a disposable component 161 that is replaced regularly, such as with each batch, every day, every week, or every month. In an embodiment, the mixing container 102 is empty initially when the disposable component 161 is connected to the system.

The mixing container 102 may be made of a flexible material, such as a polymer so its shape is not rigid. To provide support for the mixing container 102, it is held by a tub 106 which is sufficiently rigid to support the mixing container 102 when it is full of fluid. A leak sensor 107 is provided in the tub 106 and it detects leaks into the tub 106 while a temperature sensor 109 may also be provided in or on the tub 106 and it detects the temperature of the fluid in the mixing container 102. A warmer 104 may be provided as shown to provide heat to tub 106, but the warmer 104 may be omitted if another heater exists elsewhere in the system. Note that the concentrates 310 and 316 that will be supplied to the mixing container 102 may be used for making any type of medicament, not just dialysis fluid.

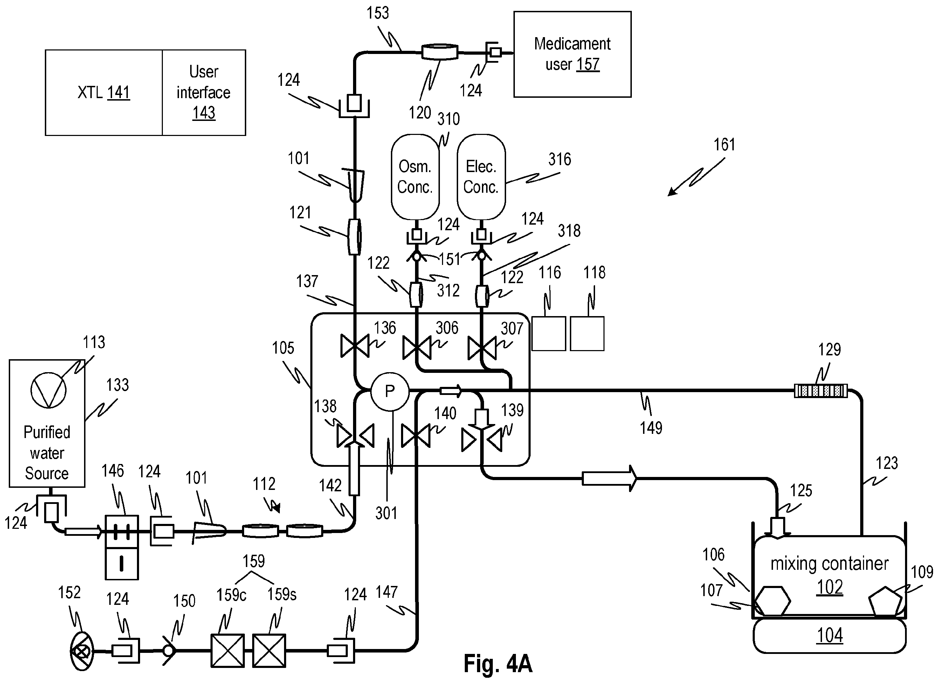

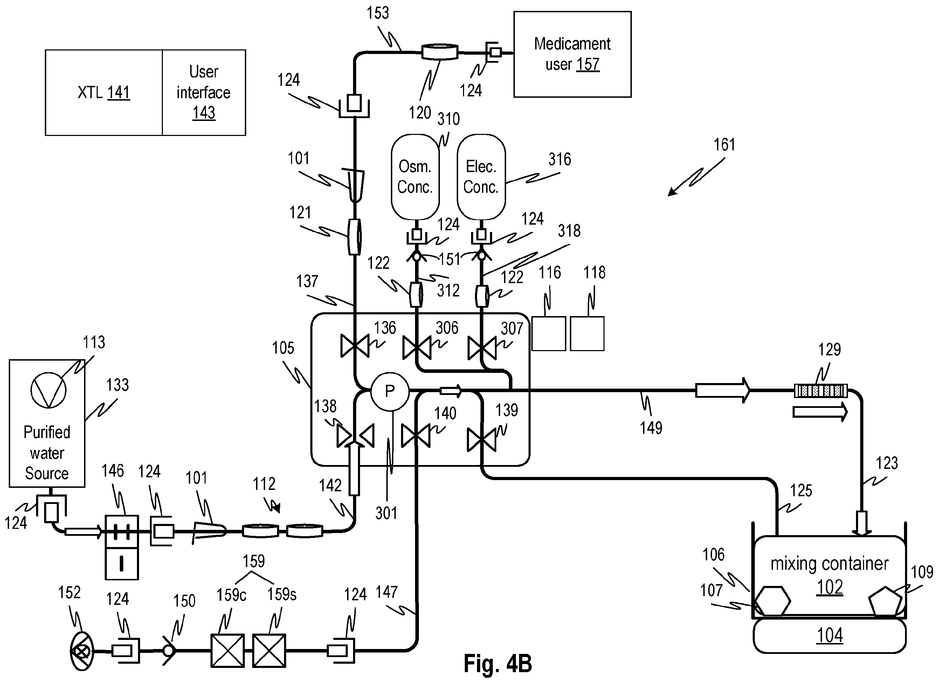

To supply water to mixing container 102, clamp 139 can remain closed, and pump 129 runs to move the water from water line 142 to supply line 123 and mixing container 102 while valve 138 is open, as shown in Fig. 4B. In embodiments, such as shown in Fig. 4A, supply line clamp 139 may be opened and the water source pump 113 operates to convey water through inlet line 125 into mixing container 102 without the use of peristaltic pump 129. Also, to make the medicament available to the medicament user 157, clamps 136 and 139 can be opened and the other clamps can be closed and the medicament pump 115 may draw from the mixing container 102 without the assistance of a predefined backpressure, hence without the use of peristaltic pump 129. Alternatively, the peristaltic pump 129 may be run through a circulating path of 149, 123, and 125 with a feedback-controlled clamp 139 according to pressure indicated by pressure sensor 301. Here clamps are closed except for 136 and 139 and the medicament user draws from a pressurized line.

Two conductivity/temperature sensors 159c and 159s are positioned on the drain line 147, beyond connector 124, but it will be understood that a single conductivity/temperature sensor 159 may include two conductivity sensors 159c and 159s. The conductivity/temperature sensor 159 is positioned on a portion of aligned switches connectable by connector 124, such that when the disposable component 161 is replaced, the conductivity/temperature sensor 159 remains and is reused.

The mixing container at 102 may be part of a disposable unit 161. Included in a disposable unit 161 are the two concentrate supply lines 312 and 318, transfer line 149, water source line 142, drain conductivity line 147, medicament supply line 153 and the mixing container 102 with its respective fill lines 123 and 125. The disposable unit 161 is permanently interconnected up to and including an end of each of the connectors 124, through which various other components can be connected (including the medicament user 157, the purified water source 133, the osmotic agent concentrate 310, the electrolyte concentrate 316, and the drain connection 152). Also included in the disposable unit 161 may be a check valve 154 that has a predefined cracking pressure (e.g., 3.5 PSI (24,131.65 Pa)). The disposable unit 161 can be connected to check valve 150 which prevents back flow in the drain conductivity line 147.

A door lock 116 is provided adjacent a user interface door 105 to lock the user interface door. A physical door 105 that opens encloses and provides access to the interior of the fluid preparation system may have a user interface on it which may be a part of user interface 143. A door sensor 118 detects whether the door lock is in an open or a locked position to ensure that all clamps and the peristaltic pump actuators are fully engaged with the disposable fluid circuit.

The door sensor 118 may include a plunger which is pressed in when the door is closed and outputs an electrical signal to indicate whether or not the door is closed. In other embodiments, the door sensor 118 may include a magnetic reed switch which detects the presence or the absence of a magnet which is located on the door 105 at a location which is detectable by the reed switch. Purified water flows into the disposable circuit where a pair of 0.2-micron filters (also in the disposable unit 161) are located to ensure that any touch contamination is prevented from flowing into the disposable circuit. An optional sterilizing filter 120 may be provided in a user medicament supply line 153. The mixing container 102 of the disposable unit 161 may have sufficient volume for a single treatment or in embodiments, multiple treatments. To make a batch of dilute concentrate, water is pumped into the mixing container 102 which contains concentrate sealed in it as delivered.

The medicament output line 137 may include an optional air removal filter 121. The air removal filter 121 may be a 1.2 µm filter which removes air.

A check valve 150 in drain conductivity line 147 ensures the flow does not reverse to safeguard against contamination in the medicament or water lines or other sterile fluid circuits. Note that the peristaltic pump 129 is regulated to ensure the output pressure remains below the cracking pressure of the check valve 154 when the conductivity of the mixing container contents is measured.

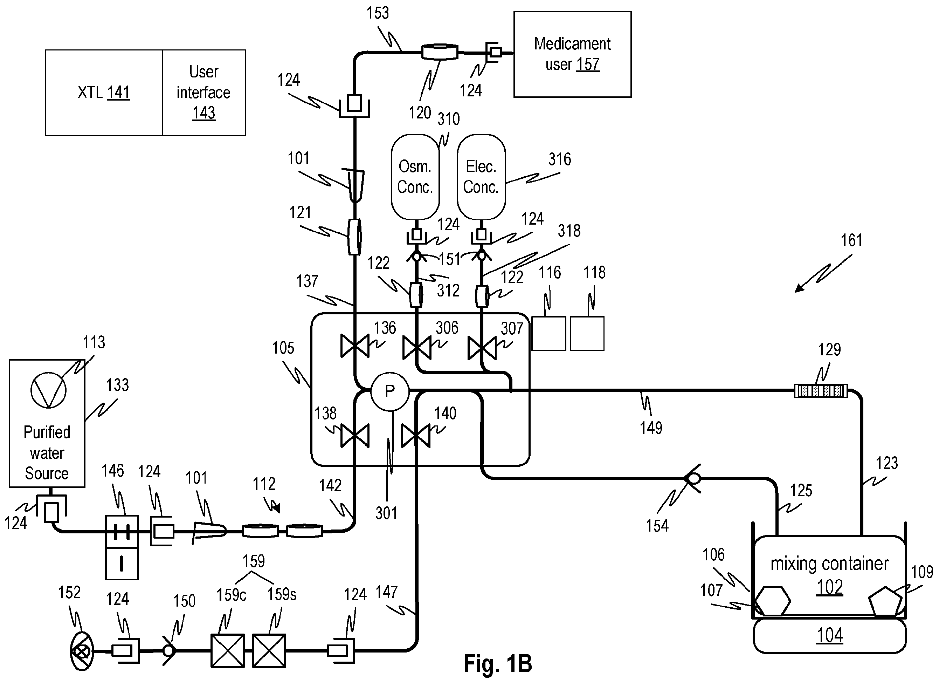

Fig. 1B shows a medicament generation system that is like that of Fig. 1A except that there is no valve 139 and instead cracking pressure check valve 154 is provided. The check valve 154 prevents flow in line 125 out of mixing container 102 and allows flow into mixing container 102 only when the cracking pressure is overcome. The cracking pressure may be selected at 3.5 PSI (24,131.65 Pa) in embodiments. As described in greater detail below, using the check valve 154 allows for different fluid line configurations.

Likewise, a check valve 151 may be added to the concentrate supply lines 312 and 318 as shown, preventing back flow of concentrate into the containers 310 and 316. In embodiments, this allows for the safe preparation of multiple batches of diluted medicament from the same containers of concentrate, as back flow (which is undesirable) into the concentrate container is prevented.

Note that in variations of most of the embodiments, the purified water source 133 may include a container or containers of purified water such as one or more polymer bags. In such embodiments, there may be a water pump arranged in a "pull" configuration. In any of the embodiments, the medicament user 157 may include a pump 115. For example, the medicament user 157 may include a dialysis cycler that is configured to draw from a container of dialysis fluid.

To permit the medicament user 157 to draw medicament on-demand, the controller may be programmed to maintain a constant pressure that is compatible with a pump in the medicament user 157. For example, the pressure-based control using the pressure sensor 301 may maintain a pressure that mimics a simple container that allows the medicament user 157 to draw from a container of dialysis fluid.

In embodiments, the medicament user 157 can use its own pump, such as the pump 115, to move fluid from the mixing container 102 without the use of pump 129. In this example, valves 136 and 139 will be opened, and the medicament user 157 will operate its pump to draw fluid form the mixing container 102.

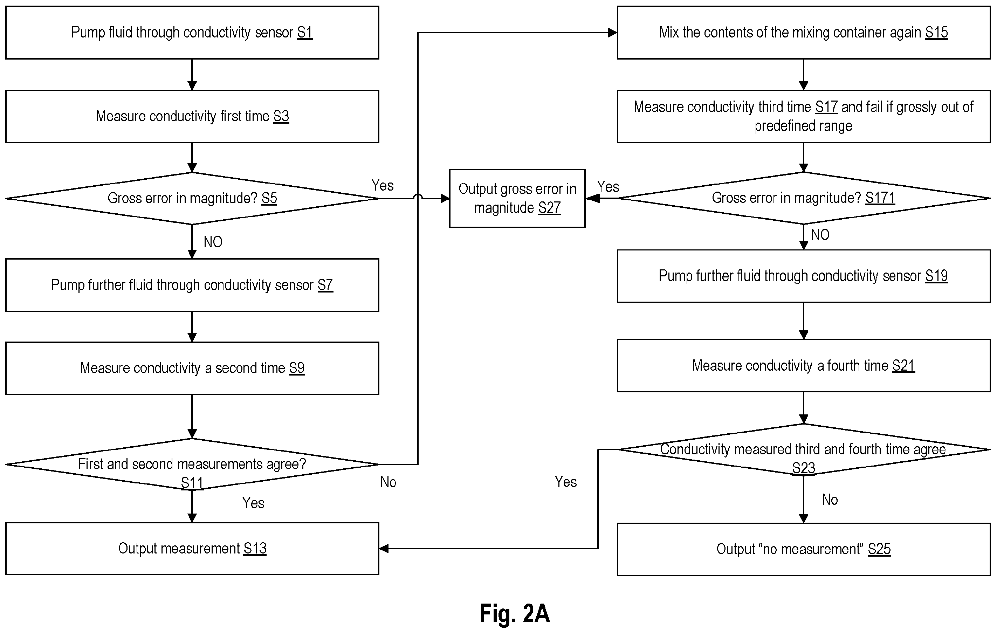

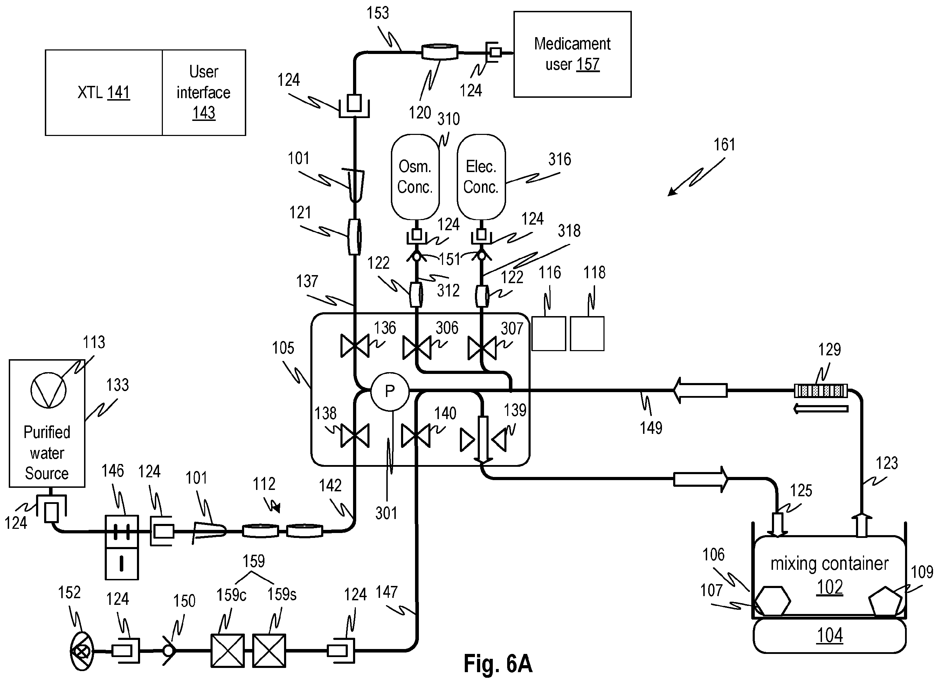

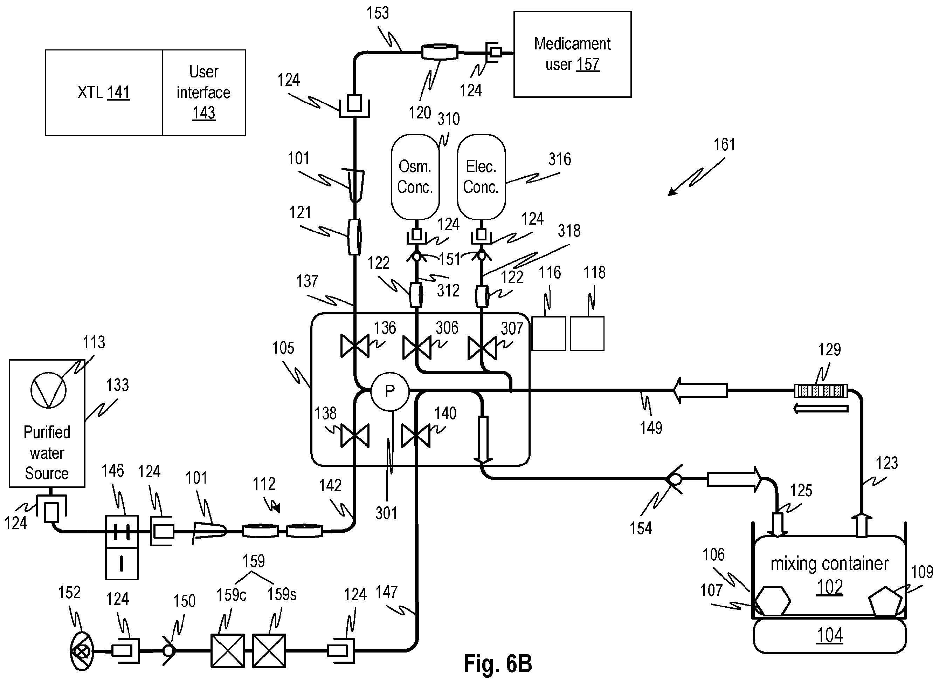

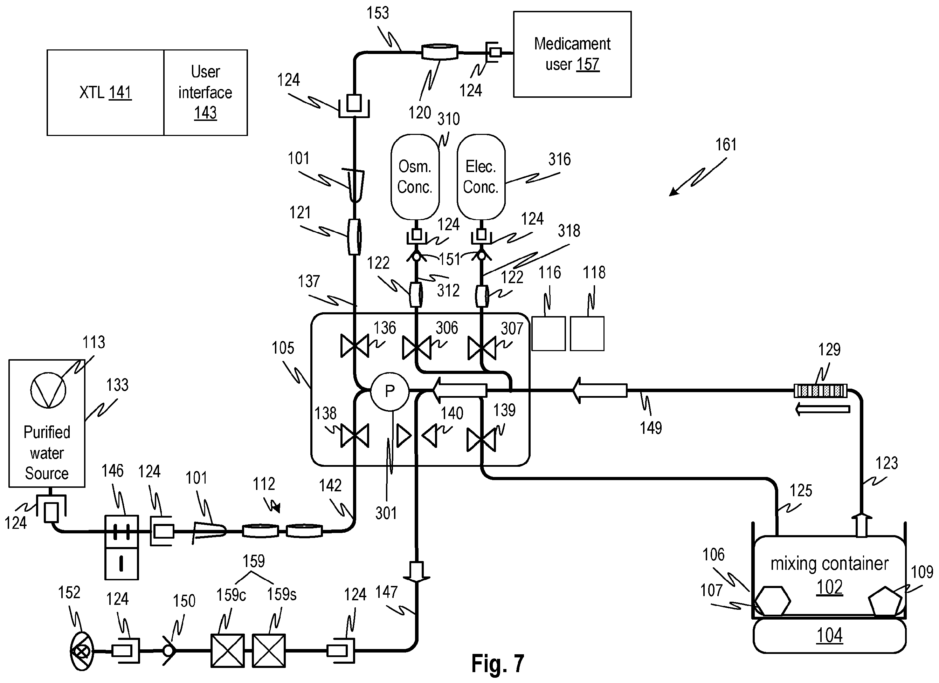

Fig. 2A shows a procedure for reliably measuring the conductivity of a fluid. The fluid circuit will be configured as shown in Fig. 7. In this procedure two consecutive measurements are made of conductivity and temperature at different times so that the conductivity is measured for two different parts of a flow stream. The two consecutive measurements can be made with a single sensor 159 at two different times, or they may be made using two different sensors such as 159c and 159s. If the two different readings are within a predefined range of each other, the controller 141 mixes the mixing container 102 a second time. The measurements are compared again and if the two conductivities are within a predefined range of each other, the measurement is output as correct. If the two measurements show a difference in concentration beyond the predefined range, then the mixing container 102 is mixed again (configuration of Fig. 6A and 6B) and two consecutive measurements are taken again. The contents of drain line 147 may be purged to the drain. The rationale behind this is that a difference in magnitude of the consecutive measurements may be caused by inadequate mixing. If, after mixing again and repeating the two consecutive measurements, the magnitudes are still outside of the predefined range of each other, then the controller outputs a measurement failure or data indicating "no measurement." Also, after the initial measurement the controller determines if there is gross disparity between the measurement and a predefined or calculated estimate then the algorithm will immediately output an indication and stop the process.

Mixed fluid is pumped through temperature and conductivity sensors 159c and 159s and is determined to be mixed when two consecutive measurements of the conductivity of mixed fluid flowing through the temperature and conductivity sensors 159c and 159s are within a predefined range of each other. If they differ by a margin greater than the predefined range, the mixing container 102 may be mixed again. An attachment to a drain or waste container is provided by a connector 152. Note the mixing bag may contain a liquid or dry concentrate which forms part of the disposable unit 161.

Referring to Fig. 2A, at S1, the fluid whose conductivity is to be measured is pumped through conductivity/temperature sensors 159c and 159s by opening the conductivity sensor clamp 140 and closing the others, as shown in Fig. 7. At S3, the peristaltic pump 129 is run in a direction indicated by the arrows as shown in Fig. 7. The conductivity is measured a first time by flowing mixed fluid from the mixing container 102 through the temperature and conductivity sensors 159c and 159s (or single conductivity sensor 159, depending on the configuration of the system) and storing a magnitude or multiple magnitude readings thereof. If the absolute value of the difference between the measured conductivity readings is greater than a predefined magnitude at S5, then control goes to S27 where an error indication is output. Otherwise, at S7, additional fluid is pumped from the mixing container 102 and at S9, the conductivity is measured a second time at S9. At S11 it is determined if the first and second measurements agree within a predefined range. If the measurements differ less a than predefined range, then the measurement is output at S13 where the output measurement may be one of the first and second measurements or an average of the measured values. If the measurements differ by more than the predefined range, then control proceeds to S15 where the mixing container contents are mixed again (because it is assumed that the measurements may differ due to insufficient mixing such that the medicament is not yet uniformly mixed in the mixing container 102). At S17, a third measurement for the conductivity is obtained. If the measured conductivity differs from the expected conductivity by a predefined magnitude at S171, a gross error is detected at S27. Otherwise, the process continues at S19, where the mixing container contents are again pumped through the conductivity sensors 159c and 159s and a fourth measurement of conductivity is made at S21 in the manner described above. At S23 it is determined if the third and fourth measurement are within the predefined range and if so, at S25, the measured values (average of the two sensors or one of them) are output at S13 as a valid conductivity measurement. If the measured values still disagree by the predefined amount, then at S25 a failure is output.

Note that the consecutive measurements may be done sequentially in time using one temperature-compensated conductivity measurement indicated by conductivity/ temperature sensor 159c, only. The fluid then is conveyed, and a temperature-compensated conductivity measurement is measured again by the same sensor 159c. In alternative embodiments, separate pairs or single temperature-compensating may be separated along a line and the measurement generated by them may be compared instead.

Note that temperature-compensated conductivity is intended to refer to a number that is proportional to concentration and may be determined in various ways including but not limited to a lookup table and a formula. For the remainder of this disclosure a reference conductivity the reference may be understood to mean temperature-compensated conductivity or an actual calculation of concentration. That is, the temperature-compensated conductivity may be a value that is generated by the controller by multiplying the measured conductivity with a value that represents the rate of change of concentration with temperature. In other embodiments, the controller 141 may calculate a concentration directly using a look-up table or formula.

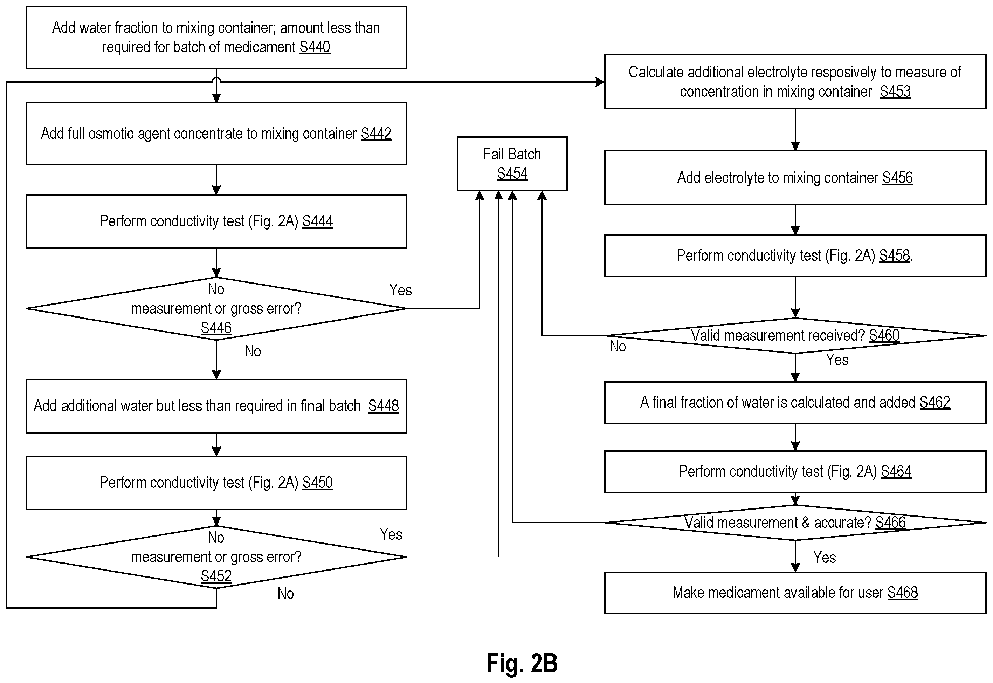

Fig. 2B shows a flow chart for a procedure that may be executed by the controller 141 with respect to the embodiment of Fig. 1A and 1B to generate medicament. It incorporates the procedure of Fig. 2A by the reference to "conductivity test" described with reference to the procedure of Fig. 2A. When the conductivity test is referenced it means the procedure of Fig. 2A is entered and upon exiting proceeds to the next procedure element in Fig. 2B.

At S440, water is added to the mixing container 102 in an amount that is a fraction of what is determined (or expected) to be required for a complete batch of medicament. The amount of fluid conveyed at S10 may be a fraction of the total estimate required for a sufficient level of dilution, such as 50% of the expected total water volume.

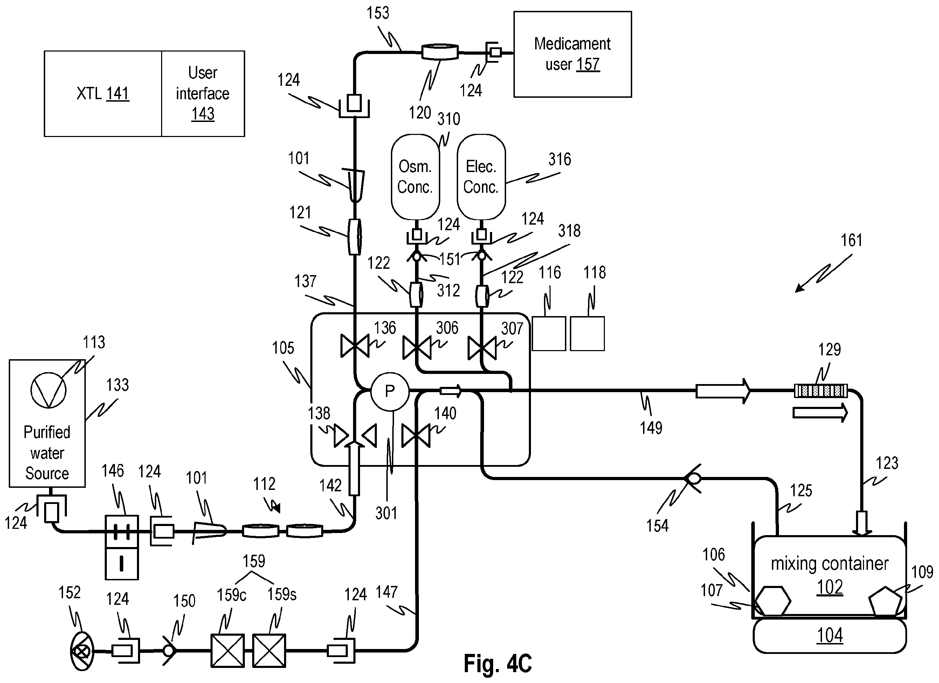

Water is added by pumping it into the mixing container 102 from the purified water source 133. This is done by placing the system in the configuration of Fig. 4A, 4B, or 4C. The water pump 113 and the peristaltic pump 129 are activated for a predefined number of cycles or a predefined time interval, resulting in a quantify of water being conveyed along water line 142, through opened valve 138, through transfer line 149, through peristaltic pump 129 and through connector line 123 into mixing container 102.

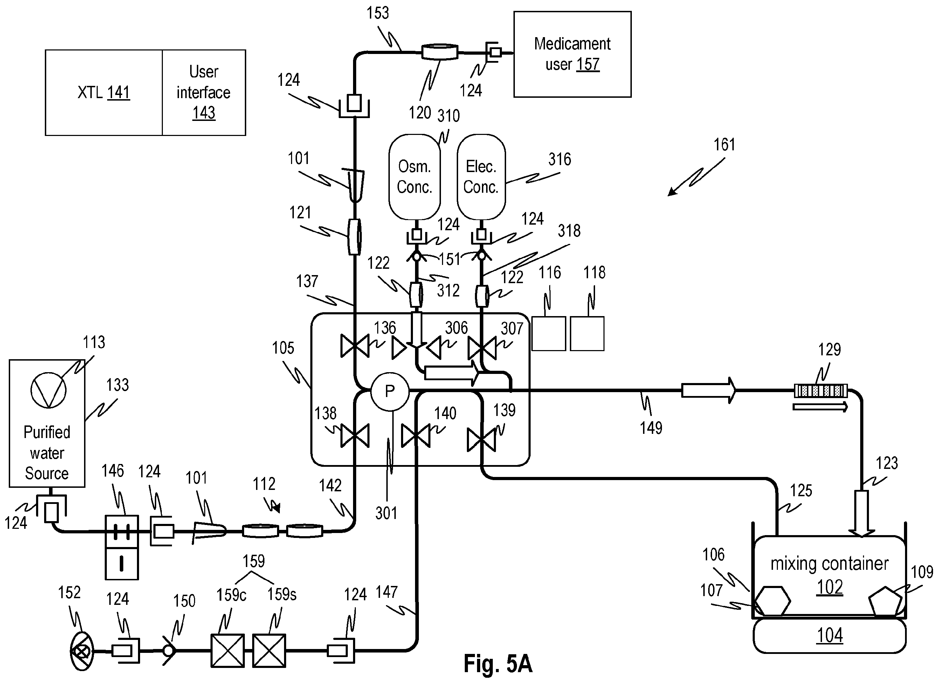

In an embodiment, the entire quantity of osmotic agent concentrate is transferred from container 310 to the mixing container 102 at S442. The fluid circuit takes on the configuration shown in Fig. 5A by controlling valve 306 to open and operating the peristaltic pump 129 in the forward direction as shown by the arrow under the pump 129 in Fig. 5A. Then the contents of the mixing container 102 are mixed by placing the fluid circuit into the configuration shown in Figs. 6A or 6B. In other embodiments, less than the entire quantity of osmotic agent concentrate from container 310 is conveyed to the mixing container 102, leaving a quantity of the concentrate in the container 310 sufficient for making additional batches of dialysate in the future.

The conductivity test described above and illustrated in Fig. 2A is then performed at S444. If an output of gross error or no measurement is received at S446, then the batch is failed at S454. If a measurement is output, control proceeds to S448 and additional water is added to the mixing container 102 short of the final amount required to achieve a batch that is usable for the medicament, and the contents of the mixing container 102 are mixed again as described above.

The conductivity test is performed again at S450 and if an output of gross error or no measurement is received S452 then the batch is failed at S454.

Otherwise, an amount of electrolyte is calculated, based on the conductivity measurement received, is at S453. Because the osmotic agent and the electrolyte concentrate are provided in separate containers 310 and 316, it is possible to generate customized batches of medicament (e.g., dialysate) based on a prescription that is customized for a specific patient. It is also possible to generate smaller quantities of diluted medicament than in a situation where all of the concentrated medicament were to be used at once, which allows for a fast walkup time (e.g., less than 1 hour) so a patient can initiate preparation of medicament and then begin therapy in less than an hour.

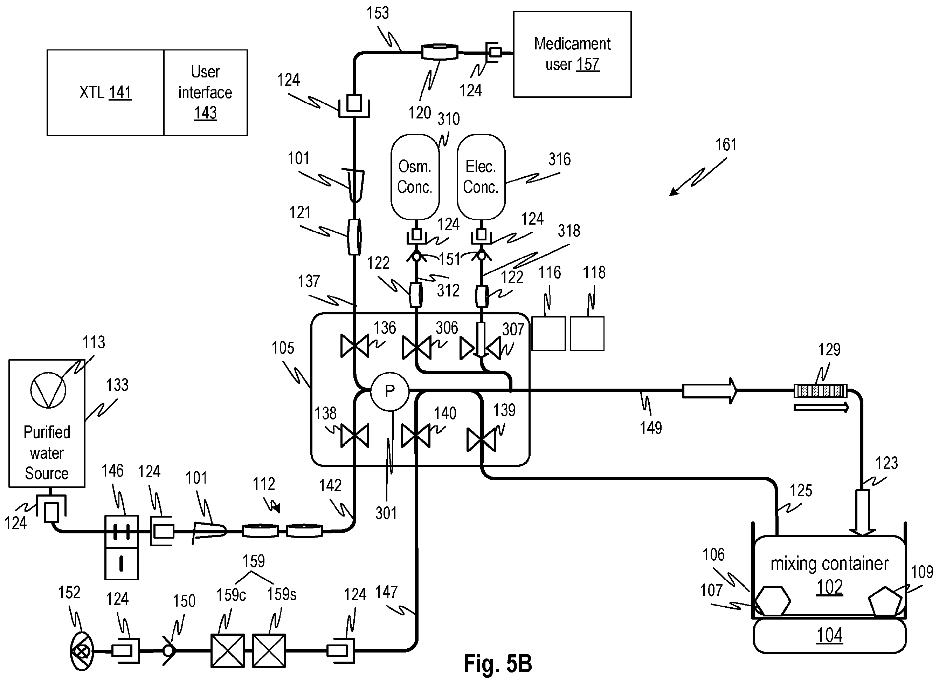

After the calculation, the appropriate amount of electrolyte concentrate is added to the mixing container 102. The fluid circuit is placed into the configuration illustrated in Fig. 5B. As shown in the figure, valve 307 is opened and the peristaltic pump 129 operates in the forward direction to convey the electrolyte concentrate into mixing container 102. It will be appreciated that because the same pump 129 is used for metering both the osmotic agent concentrate from container 310 and the electrolyte concentrate from container 316, the accuracy of the metering is increased, allowing for high precision in establishing the desired custom concentration of the medicament. Once all of the electrolyte concentrate is added, the contents of the mixing container 102 are mixed again as described above.

At S458 the conductivity test is performed again and if a valid measurement is not received at S460, then the batch is failed at S454. If the measurement is received, then at S462 a final fraction of water is then calculated based on the valid measurement and added to the mixing container 102 by placing the fluid circuit into a configuration as shown in Figs. 4A or 4B. Then, the contents of the mixing container 102 are mixed again as described above.

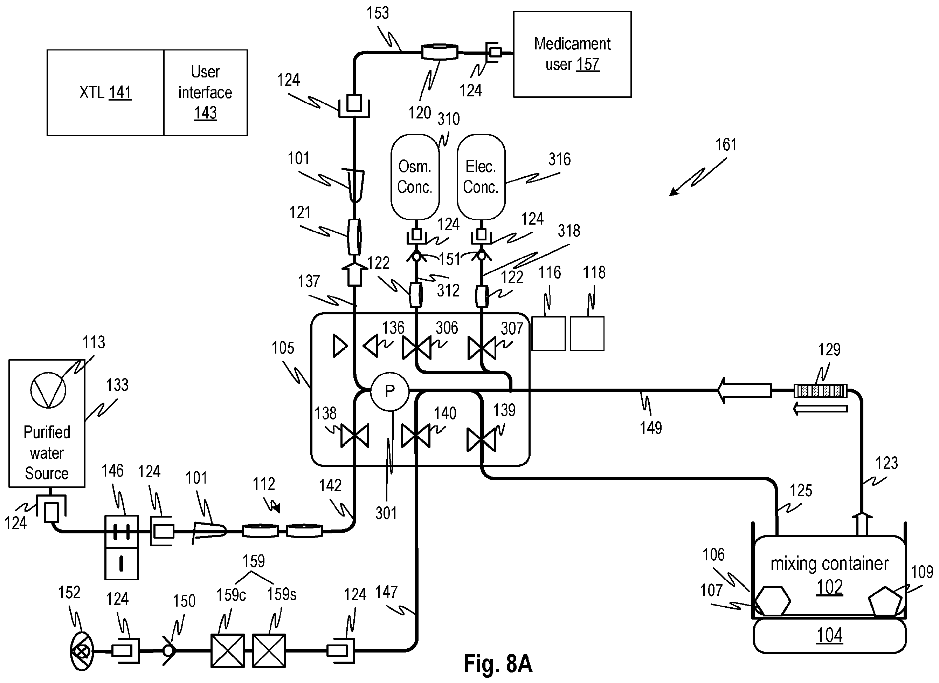

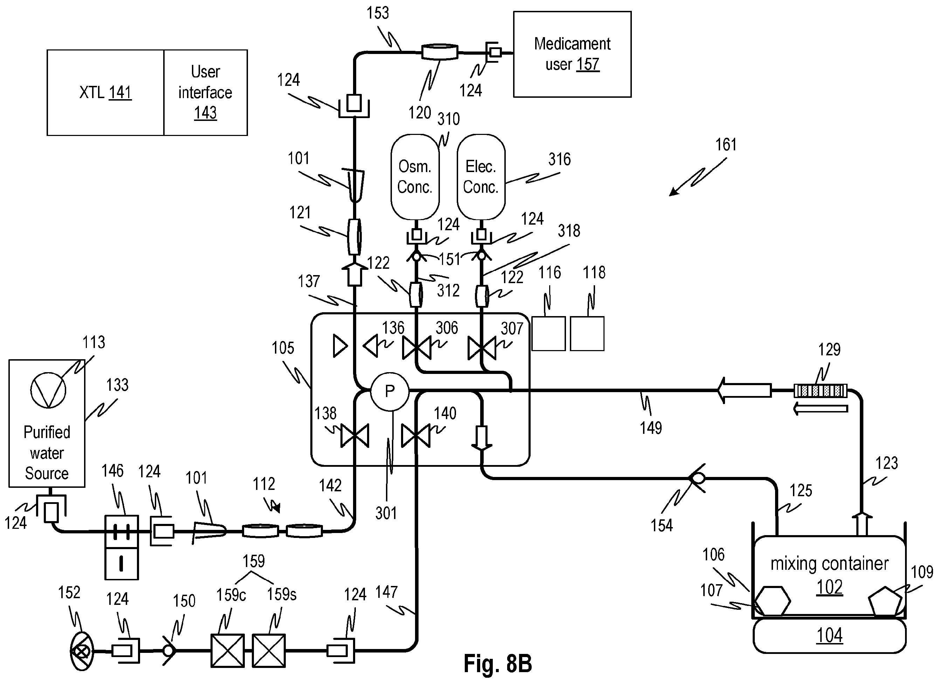

The conductivity test is performed again at S464. If the measurement is valid at S466, then the batch is made available for use at S468. Otherwise, the batch is failed at S454. When the batch is made available, the fluid circuit is configured into the configuration shown in Figs. 8A or 8B, and described below.

Note there may be a single conductivity/temperature sensor, or a pair of conductivity/temperature sensors as shown. A pair of conductivity/temperature sensors may provide a check against poor accuracy or failure of one of the sensors. During the conductivity check, the fluid from the mixing container flows through the drain conductivity line 147 using the peristaltic pump 129.

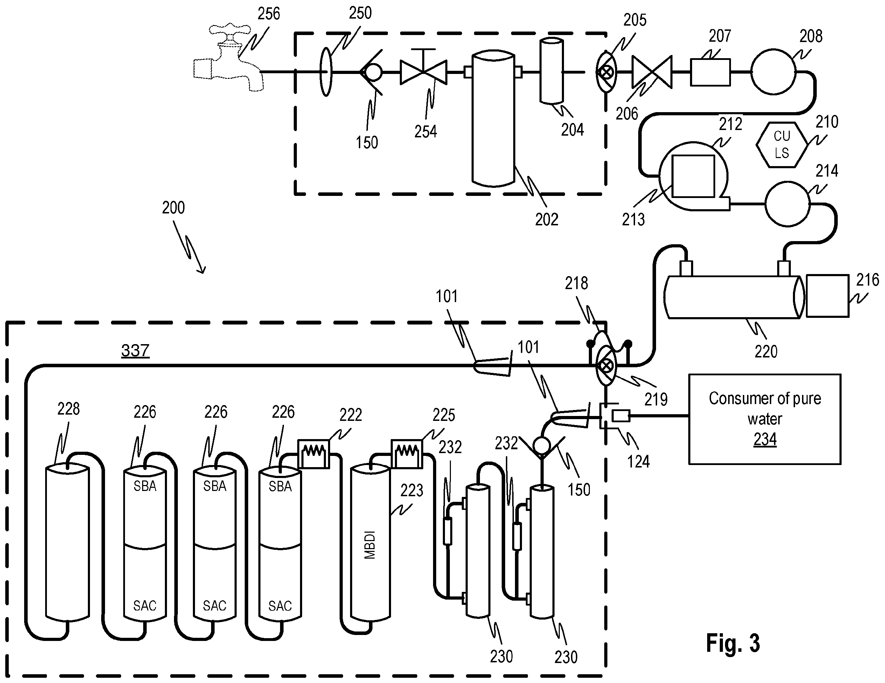

Fig. 3 shows a water treatment plant 200 that may constitute an embodiment the purified water source 133. The water treatment plant 200 has an initial pretreatment stage that includes a connector 250 to connect to an unfiltered water source 256, for example a water tap. The water flows through a check valve 150, through a pressure regulator 254, and then through a sediment filter 202. The check valve 150 prevents backflow of the water. The water then flows through an air vent 204 that removes air from the water. The water then flows through a connector 205 that connects to a water shutoff clamp 206, a snubber 207, and a water inlet pressure sensor 208. Water is pumped by water pump 212 which has an encoder 213 for precise tracking of the water pump 212 speed. The snubber 207 reduces pressure fluctuations. The water then flows through a water output pressure sensor 214, through an ultraviolet light lamp 220 and into a filter plant 337 that performs deionization, carbon filtration, and sterilizing filtration. A UV (ultra violet) light sensor 216 may be provided to detect whether the ultraviolet light lamp 220 is operating, so that it can be replaced if it becomes inoperable. A first-use-fuse 218 together with a connector 219 is provided on the inlet of sterilizing filter plant 337, such that the fuse indicates whether the filter plant 337 has been used. This helps reduce the likelihood that a previously-used filter plant is reused unintentionally. A combined control unit and leak sensor are indicated at 210. In the sterilizing filter plant 337, the water flows through a carbon filter 228 and three separated bed deionization filters 226 which may be resin separated bed filters. The water then flows through a mixed bed deionization filter 223, which follows the separated bed filters 226. The mixed bed deionization filter 223 may be a resin mixed bed filter. Thereafter, the water flows through first and second ultrafilters 230, which follow the mixed bed deionization filter 223, and into the consumer of pure water 234. The embodiments of Figs. 1A and 1B are examples of a consumer of pure water 234.

Between a last separated bed deionization filter 226 and the mixed bed deionization filter 223 is a resistivity sensor 222 which indicates when the separated bed deionization filters 226 are nearing exhaustion, or at exhaustion. The resin mixed bed deionization filter 223 is still able to hold a predefined minimum magnitude of resistivity but the deionization resin separated bed filters 226 and the mixed bed deionization filter 223 may be replaced at the same time. In embodiments, along with the separated bed deionization filters 226 and the mixed bed deionization filter 223, the carbon filter 228 and ultrafilters 230 along with the interconnecting lines and other components may also be replaced as a single package. A current treatment can be completed in reliance on the mixed bed deionization filter 223 before the exhausted filters are replaced. A further resistivity sensor 225 detects unexpected problems with the separated bed deionization filter 223 upstream deionization filters which may require shutdown of the treatment and immediate replacement of the filters. Note that each of the ultrafilters 230 has an air vent 232. A check valve 150 is located downstream of the ultrafilters 230. The consumer of pure water 234 may be unit such as that of Figs. 1A or 1B which mixes a batch of medicament for use by a medicament user 157 such as a peritoneal dialysis cycler or any other type of medicament consuming device.

It should be evident from the above that the procedures of Fig. 2B in combination with those of Fig. 2A may be performed using the embodiments of Figs. 1A or 1B.

Note in any of the embodiments where the term clamp is used, it should be recognized that the functional element includes a tube or other flexible conduit and the clamp so that it functions as a valve. In any of the embodiments, another type of valve may be substituted for the clamp and conduit to provide the same function. Such a variation may be considered to alternative embodiments and clamp and conduit are not limiting of the subject matter conveyed herein.

Note that in any of the embodiments that identify the bag as the container, any bag may be replaced by any container including those of glass, polymer and other materials. In any embodiment where flow control is performed by a clamp, it should be understood that in any embodiment, any clamp can be replaced by another type of valve such as a stopcock valve, a volcano valve, a ball valve, a gate valve or other type of flow controller. It should be understood that a clamp in the context of the disclosed subject matter is a clamp that closes around a tube to selectively control flow through the position of the clamp. Note that in any of the embodiments, the order of adding and mixing to the mixing container 102 can by reversed from what is described with respect to the embodiments. In any of the embodiments instead of dextrose concentrate being used, this can be substituted for glucose or another osmotic agent.

The process of providing purified water from the purified water source 133 is described next. As shown in Fig. 4A, water inlet clamp 138 is opened and the water pump 113 operates to convey purified water along water line 142. Valve 139 can be opened so that the water pump 113 alone, without the involvement of peristaltic pump 129 conveys water into the mixing container 102 through line 125. Alternatively, valve 139 can be closed and peristaltic pump 129 operates to move water from water supply line 149 to inlet line 123 and through the inlet line 123 into mixing container 102. In this situation, pump 113 provides a positive upstream pressure for the peristaltic pump 129, as shown in Fig. 4B.

In an alternative embodiment, as illustrated in Fig. 4C, clamp 139 is not present but the two pumps 113 and 129 are controlled such that the water pressure in the line is below the cracking pressure of the check valve 154 in the embodiment of Fig. 1B. This way, the water enters the mixing container only through supply line 123. On the other hand, in the embodiment of Fig. 1A and 1C, the additional valve 139 can be closed to ensure that water does not flow through supply line 125. Note that valve 139 is not present in the embodiment of Fig. 1B. Further, the pumps 113 and 129 are controlled by controller 141 to provide a consistent upstream pressure for the peristaltic pump 129.

Referring to Figs. 4B and 4C, water is provided from the purified water source 133 to the system. The peristaltic pump 129 is configured to move fluid in the line 123 connected to the mixing container 102. The peristaltic pump 129 also moves fluid, at selected times, through the line 125 which returns the fluid to the mixing bag. Line 125 can be provided with the check valve 154 (Fig. 1B) which prevents flow in one direction and has a cracking pressure which must be overcome for water to flow in the other direction. In the example of Fig. 1B, the check valve permits water to flow through line 125 toward the mixing container 102 when the cracking pressure of the check valve 154 is overcome. Initially the purified water from the purified water source 133 is pumped by the water pump 113 with water inlet clamp 138 open and the batch release clamp 136 and the conductivity sensor clamp 140 closed such that water is pumped into the mixing container 102 through line 123 with the peristaltic pump 129 running so as to convey water into the mixing container 102, as shown in Figs. 4B and 4C.

Fig. 5A illustrates the configuration of the system when the osmotic agent concentrate 310 (e.g., dextrose, glucose, etc.) flows through osmotic supply line 312 and eventually into the mixing container 102. As shown in the figure, valve 306 is opened and peristaltic pump 129 can operate in in the direction shown (pictured to the right on the drawing sheet), such that the osmotic concentrate 310 flows through inlet line 123 into mixing container 102. The peristaltic pump 129 can be controlled to precisely meter a desired quantity of the osmotic concentrate into mixing container 102. In embodiments, only a fraction of the total quantity of the osmotic agent concentrate 310 present in its container is provided into mixing container 102, such that multiple batches of the medicament can be prepared in the mixing container 102; and each of the batches can be customized based on a desired concentration to create custom mini batches.

In an alternate embodiment, the osmotic concentrate 310 can be positioned sufficiently high or above mixing container 102 that a gravity powered fill can be accomplished. In this scenario, valve 306 is opened and valve 139 is opened (not illustrated in Fig. 5A) which permits gravity to convey the osmotic agent concentrate through inlet line 125 into mixing container 102, without the use of peristaltic pump 129. In embodiments, the entirety of the osmotic agent concentrate 310 is allowed to flow into the mixing container 102 so that the quantity of the osmotic agent concentrate 310 that is present in the mixing container 102 is known based on the original amount of the osmotic agent concentrate that was present in its initial container.

Fig. 5B illustrates the configuration of the system when the electrolyte concentrate 316 flows through electrolyte supply line 318 and eventually into the mixing container 102. As shown in the figure, valve 307 is opened and peristaltic pump 129 can operate in in the direction shown (pictured to the right on the drawing sheet), such that the electrolyte concentrate 316 flows through inlet line 123 into mixing container 102. The peristaltic pump 129 can be controlled to precisely meter a desired quantity of the electrolyte concentrate into mixing container 102. In embodiments, only a fraction of the total quantity of the electrolyte concentrate 316 present in its container is provided into mixing container 102, such that multiple batches of the medicament can be prepared in the mixing container 102; and each of the batches can be customized based on a desired concentration to create custom mini batches.

In an alternate embodiment, the electrolyte concentrate 316 can be positioned sufficiently high or above mixing container 102 that a gravity powered fill can be accomplished. In this scenario, valve 307 is opened and valve 139 is opened (not illustrated in Fig. 5B) which permits gravity to convey the electrolyte concentrate through inlet line 125 into mixing container 102, without the use of peristaltic pump 129. In embodiments, the entirety of the electrolyte concentrate 316 is allowed to flow into the mixing container 102 so that the quantity of the electrolyte concentrate 316 that is present in the mixing container 102 is known based on the original amount of the electrolyte concentrate that was present in its initial container.

Referring to Fig. 6A and 6B, to mix the contents of the mixing container 102 the peristaltic pump 129 pumps fluid in a circular path through lines 123, 125, and 149 in a direction designated with an arrow in the figure (to the left in the figure) with all the clamps closed except for clamp 139 in Fig. 6A. In the embodiment of Fig. 6B, there is no clamp 139, so the peristaltic pump 129 generates sufficient pressure to overcome the cracking pressure of check valve 154. Then the contents of the mixing container 102 are mixed by the flow circulating through the mixing container 102.

Referring to Fig. 7, after a sufficient time of mixing, a sample of the fluid in the mixing container 102 may be pumped through a drain conductivity line 147 which contains conductivity/temperature sensors 159c and 159s (control sensor 159c and safety sensor 159s) to determine a temperature-compensated conductivity of the diluted medicament. Each sensor 159c and 159s may be configured to calculate conductivity and temperature of fluid passing through or past the sensor. Two redundant sensors 159c and 159s may be provided, to enable a comparison of their respective measurements and thereby to confirm that the sensors are functioning. If their respective measurements are within a predetermined range, the sensors are understood to be functioning correctly. On the other hand, if their respective measurements are outside of the predetermined range, an error condition may be signaled as described below. Although two separate sensors 159c and 159s are shown, a single sensor 159 may be provided instead, and multiple readings may be taken over time to generate multiple values for comparison and to determine the proper functioning of the sensor 159.

Valve 140 is opened and the peristaltic pump 129 operates in reverse direction as shown in the figure to covey fluid from the mixing container 102 toward the conductivity sensor(s) 159. A check valve 150 prevents backflow of fluid from the drain connection 152. In Fig. 7, the fluid channel that contains the conductivity sensor(s) 159 may be provided with connectors 124, as shown, so that the whole fluid channel can be replaced as needed if the accuracy of the conductivity sensor(s) 159 degrades or indicates a failure.

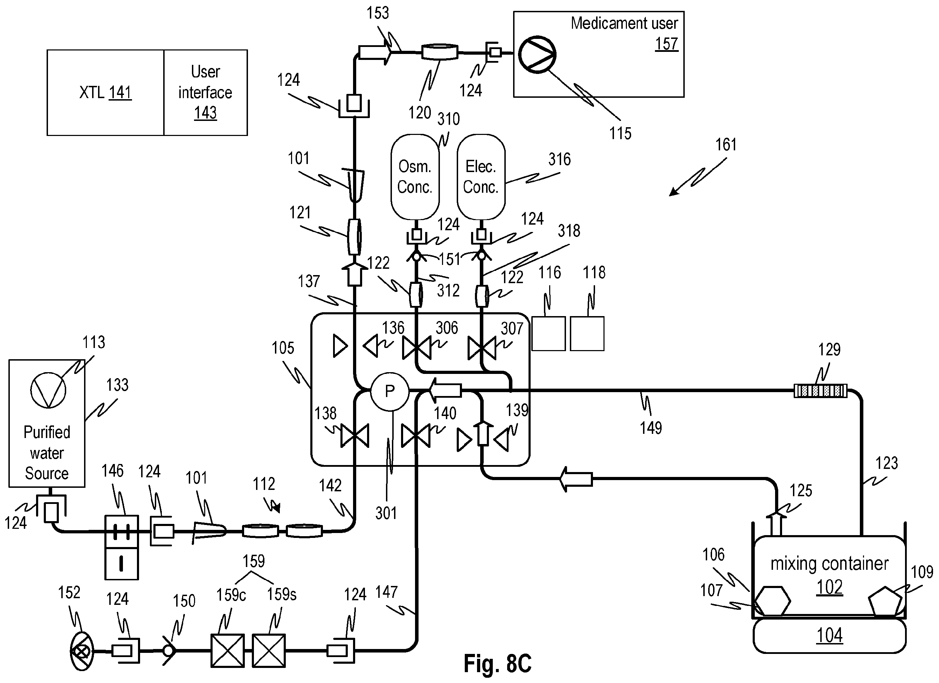

Referring now to Fig. 8A, 8B, and 8C, once of the medicament is prepared and mixed in the mixing container at 102, and the medicament is deemed to be ready for use based on conductivity checks described above, the medicament is provided to the medicament user 157. Figs. 8 A-C illustrate various arrangements of the fluid circuit for providing the medicament. At this time, the water inlet clamp 138 and the conductivity sensor clamp 140 are closed. The medicament user 157 may be any type of treatment device or container that receives the mixed medicament from the mixing container 102.

The batch release clamp 136 is open and the water inlet clamp 138 and the conductivity sensor clamp 140 are closed. The pump 115 in the medicament user 157 may then draw fluid from the circular path as the peristaltic pump 129 rotates to maintain fluid at the cracking pressure of the check valve 154 in Fig. 8B, or at a pressure that is controlled based on a pressure signal from pressure sensor 301, if no cracking check valve is used in Fig. 8A. In embodiments, the cracking pressure may be 3.5 PSI (pounds per square inch) (24,131.65 Pa). It will be understood that this makes the medicament preparation system appear like a bag of dialysate with a head pressure of 3.5 PSI (24,131.65 Pa).

The medicament pump 115 in the medicament user 157 may see a positive pressure at the cracking pressure type check valve 154 cracking pressure, which may facilitate the pump 115 of the medicament user 157 by mimicking the pressure of an elevated medicament container with a head pressure approximately at the cracking pressure of the check valve 154. In embodiments, clamp 139 is closed while peristaltic pump 129 operates in the direction shown in the drawing. Clamp 136 is opened, and the medicament is conveyed through medicament output lines 137 and 153 to medicament user 157. A pressure sensor 301 is provided to measure the pressure in this fluid channel and to provide a signal, which may be used in feedback control, to modulate the speed of the peristaltic pump 129 and thereby provide a predetermined pressure in the formed fluid channel.

In further embodiments illustrated in Fig. 8C, the peristaltic pump 129 is not used, and instead medicament user pump 115 operates to draw the medicament from the mixing container 102. Clamp 139 and clamp 136 are both opened, thereby providing a fluid path between the mixing container 102 and the medicament user 157. It is possible to elevate mixing container 102 to such a level that it provides a positive pressure (head pressure) for the medicament user 157.

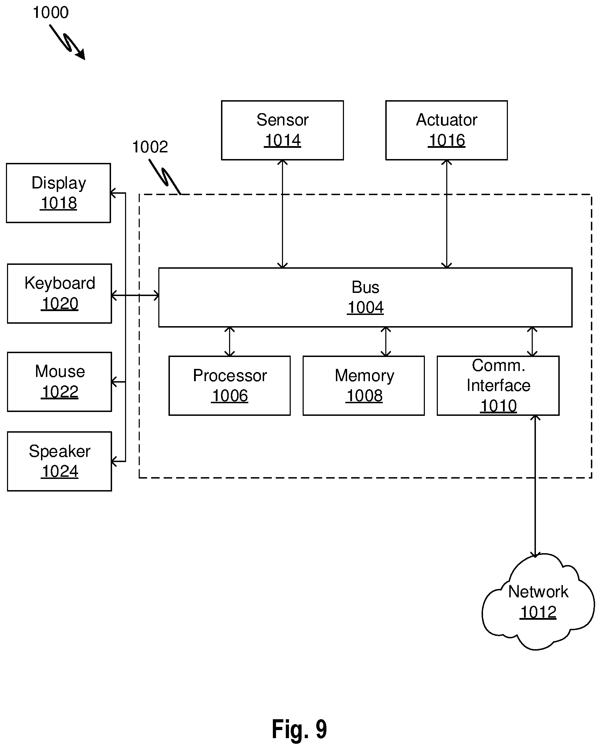

Fig. 9 shows a block diagram of an example computer system according to embodiments of the disclosed subject matter. In various embodiments, all, or parts of system 1000 may be included in a medical treatment device/system such as a renal replacement therapy system. In these embodiments, all, or parts of system 1000 may provide the functionality of a controller of the medical treatment device/systems. In some embodiments, all, or parts of system 1000 may be implemented as a distributed system, for example, as a cloud-based system.

System 1000 includes a computer 1002 such as a personal computer or workstation or other such computing system that includes a processor 1006. However, alternative embodiments may implement more than one processor and/or one or more microprocessors, microcontroller devices, or control logic including integrated circuits such as ASIC.

Computer 1002 further includes a bus 1004 that provides communication functionality among various modules of computer 1002. For example, bus 1004 may allow for communicating information/data between processor 1006 and a memory 1008 of computer 1002 so that processor 1006 may retrieve stored data from memory 1008 and/or execute instructions stored on memory 1008. In one embodiment, such instructions may be compiled from source code/objects provided in accordance with a programming language such as Java, C++, C#, .net, Visual Basic^{™} language, LabVIEW, or another structured or object-oriented programming language. In one embodiment, the instructions include software modules that, when executed by processor 1006, provide renal replacement therapy functionality according to any of the embodiments disclosed herein.

Memory 1008 may include any volatile or non-volatile computer-readable memory that can be read by computer 1002. For example, memory 1008 may include a non-transitory computer-readable medium such as ROM, PROM, EEPROM, RAM, flash memory, disk drive, etc. Memory 1008 may be a removable or non-removable medium.

Bus 1004 may further allow for communication between computer 1002 and a display 1018, a keyboard 1020, a mouse 1022, and a speaker 1024, each providing respective functionality in accordance with various embodiments disclosed herein, for example, for configuring a treatment for a patient and monitoring a patient during a treatment.

Computer 1002 may also implement a communication interface 1010 to communicate with a network 1012 to provide any functionality disclosed herein, for example, for alerting a healthcare professional and/or receiving instructions from a healthcare professional, reporting patient/device conditions in a distributed system for training a machine learning algorithm, logging data to a remote repository, etc. Communication interface 1010 may be any such interface known in the art to provide wireless and/or wired communication, such as a network card or a modem.

Bus 1004 may further allow for communication with one or more sensors 1014 and one or more actuators 1016, each providing respective functionality in accordance with various embodiments disclosed herein, for example, for measuring signals.

It will be appreciated that the modules, processes, systems, and sections described above can be implemented in hardware, hardware programmed by software, software instruction stored on a non-transitory computer readable medium or a combination of the above. For example, a method for providing a medicament to a medicament user can be implemented, for example, using a processor configured to execute a sequence of programmed instructions stored on a non-transitory computer readable medium. For example, the processor can include, but not be limited to, a personal computer or workstation or other such computing system that includes a processor, microprocessor, microcontroller device, or is comprised of control logic including integrated circuits such as, for example, an Application Specific Integrated Circuit (ASIC). The instructions can be compiled from source code instructions provided in accordance with a programming language such as Java, C++, C#.net or the like. The instructions can also comprise code and data objects provided in accordance with, for example, the Visual Basic^{™} language, LabVIEW, or another structured or object-oriented programming language. The sequence of programmed instructions and data associated therewith can be stored in a non-transitory computer-readable medium such as a computer memory or storage device which may be any suitable memory apparatus, such as, but not limited to read-only memory (ROM), programmable read-only memory (PROM), electrically erasable programmable read-only memory (EEPROM), random-access memory (RAM), flash memory, disk drive and the like.

Furthermore, the modules, processes, systems, and sections can be implemented as a single processor or as a distributed processor. Further, it should be appreciated that the steps mentioned above may be performed on a single or distributed processor (single and/or multicore). Also, the processes, modules, and sub-modules described in the various figures of and for embodiments above may be distributed across multiple computers or systems or may be co-located in a single processor or system. Exemplary structural embodiment alternatives suitable for implementing the modules, sections, systems, means, or processes described herein are provided below.

The modules, processors or systems described above can be implemented as a programmed general purpose computer, an electronic device programmed with microcode, a hard-wired analog logic circuit, software stored on a computer-readable medium or signal, an optical computing device, a networked system of electronic and/or optical devices, a special purpose computing device, an integrated circuit device, a semiconductor chip, and a software module or object stored on a computer-readable medium or signal, for example.

Embodiments of the method and system (or their sub-components or modules), may be implemented on a general-purpose computer, a special-purpose computer, a programmed microprocessor or microcontroller and peripheral integrated circuit element, an ASIC or other integrated circuit, a digital signal processor, a hardwired electronic or logic circuit such as a discrete element circuit, a programmed logic circuit such as a programmable logic device (PLD), programmable logic array (PLA), field-programmable gate array (FPGA), programmable array logic (PAL) device, or the like. In general, any process capable of implementing the functions or steps described herein can be used to implement embodiments of the method, system, or a computer program product (software program stored on a non-transitory computer readable medium).

Furthermore, embodiments of the disclosed method, system, and computer program product may be readily implemented, fully or partially, in software using, for example, object or object-oriented software development environments that provide portable source code that can be used on a variety of computer platforms. Alternatively, embodiments of the disclosed method, system, and computer program product can be implemented partially or fully in hardware using, for example, standard logic circuits or a very-large-scale integration (VLSI) design. Other hardware or software can be used to implement embodiments depending on the speed and/or efficiency requirements of the systems, the particular function, and/or particular software or hardware system, microprocessor, or microcomputer being utilized. Embodiments of the method, system, and computer program product can be implemented in hardware and/or software using any known or later developed systems or structures, devices and/or software by those of ordinary skill in the applicable art from the function description provided herein and with a general basic knowledge of control systems of medical devices and/or computer programming arts.

Moreover, embodiments of the disclosed method, system, and computer program product can be implemented in software executed on a programmed general-purpose computer, a special purpose computer, a microprocessor, or the like.

It is, thus, apparent that there is provided, in accordance with the present disclosure, Medicament Preparation Devices, Methods, and Systems. Many alternatives, modifications, and variations are enabled by the present disclosure. Features of the disclosed embodiments can be combined, rearranged, omitted, etc., within the scope of the present disclosure to produce additional embodiments. Furthermore, certain features may sometimes be used to advantage without a corresponding use of other features. Accordingly, Applicants intend to embrace all such alternatives, modifications, equivalents, and variations that are within the scope of the present disclosure. The invention is however defined by the scope of the appended claims.

## Claims

1. A non therapeutic method of generating a custom mini batch of dialysate with a proportioning system, the method comprising:
attaching a disposable component (161) to the proportioning system;
generating purified water with a water purification system (133);
adding a first quantity of the purified water to a mixing container (102) that is pre-attached to the disposable component (161);
conveying a second quantity of a first concentrated medicament to the mixing container (102);
first mixing contents of the mixing container (102);
determining a concentration of the contents of the mixing container (102);
conveying a third quantity of a second concentrated medicament to the mixing container (102);
second mixing the contents of the mixing container (102);
confirming a final concentration of the contents of the mixing container (102); and
providing the contents of the mixing container (102) to a medicament user (157), wherein
the determining the concentration of the contents of the mixing container (102) includes measuring a conductivity of the contents,
**characterized in that**
the measuring of the conductivity of the contents includes
pumping a first quantity of the contents through a conductivity sensor (159, 159c, 159s) and first measuring, by the conductivity sensor (159, 159c, 159s), a conductivity of the first quantity of the contents;
in response to determining that a magnitude of the measured conductivity of the first quantity of the contents is not greater than a predefined magnitude, pumping a second quantity of the contents through the conductivity sensor (159, 159c, 159s) and measuring, by the conductivity sensor (159, 159c, 159s), a conductivity of the second quantity of the contents;
in response to determining that the measured conductivity of the second quantity of the contents differs from the measured conductivity of the first quantity of the contents by more than a predefined range, further mixing the contents and subsequently pumping a third quantity of the contents through the conductivity sensor (159, 159c, 159s) and measuring, by the conductivity sensor (159, 159c, 159s), a conductivity of the third quantity of the contents;
in response to determining that a magnitude of the measured conductivity of the third quantity of the contents is not greater than a second predefined magnitude, pumping a fourth quantity of the contents through the conductivity sensor (159, 159c, 159s) and measuring, by the conductivity sensor (159, 159c, 159s), a conductivity of the fourth quantity of the contents; and
in response to determining that the measured conductivity of the fourth quantity of the contents differs from the measured conductivity of the third quantity of the contents by less than a predefined range, outputting a measurement based on either one or both of the measured conductivity of the third quantity of the contents and the measured conductivity of the fourth quantity of the contents.

2. The method according to claim 1, further comprising:
connecting a first source (310) of the first concentrated medicament to the disposable component (161) with a connector (124); and
connecting a second source (316) of the second concentrated medicament to the disposable component (161) with a second connector (124).

3. The method according to claim 1, further comprising:
conveying a variable quantity of the purified water to the mixing container (102) after the first mixing, wherein the variable quantity is determined based on the determined concentration of the contents.

4. The method according to claim 3, further comprising:
further determining a concentration of the contents at a time after conveying the variable quantity of the purified water to the mixing container (102) and before conveying the third quantity of the second concentrated medicament to the mixing container (102).

5. The method according to claim 1, further comprising:
conveying a second variable quantity of the purified water to the mixing container (102) after the second mixing, wherein the second variable quantity is determined based on a determined concentration of the contents of the mixing container (102) after the second mixing.

6. The method according to claim 1, wherein the providing of the contents of the mixing container (102) to the medicament user (157) takes place less than an hour after an initiation of production of the dialysate.

7. The method according to claim 1, wherein the conveying of the third quantity of the second concentrated medicament to the mixing container (102) comprises conveying the third quantity of the second concentrated medicament to the mixing container (102) in response to the determining of the concentration of the contents of the mixing container (102) indicating that there is no gross error in a measurement of the concentration of the contents of the mixing container (102).

8. The method of claim 1, wherein the first concentrated medicament is an osmotic agent concentrate and the second concentrated medicament is an electrolyte concentrate.

## Patentansprüche

1. Ein nicht-therapeutisches Verfahren zur Herstellung einer kundenspezifischen Mini-Charge von Dialysat mit einem Dosiersystem, wobei das Verfahren umfasst:
Anbringen einer Einwegkomponente (161) an dem Dosiersystem;
Erzeugen von aufbereitetem Wasser mit einem Wasseraufbereitungssystem (133);
Zugeben einer ersten Menge des aufbereiteten Wassers in einen Mischbehälter (102), der bereits an der Einwegkomponente (161) angebracht ist;
Fördern einer zweiten Menge eines ersten konzentrierten Arzneimittels zu dem Mischbehälter (102);
erstes Mischen der Inhalte des Mischbehälters (102);
Bestimmen einer Konzentration der Inhalte des Mischbehälters (102);
Fördern einer dritten Menge eines zweiten konzentrierten Arzneimittels zu dem Mischbehälter (102);
zweites Mischen der Inhalte des Mischbehälters (102);
Bestätigen einer Endkonzentration der Inhalte des Mischbehälters (102); und
Bereitstellen der Inhalte des Mischbehälters (102) für einen Arzneimittelanwender (157), wobei
das Bestimmen der Konzentration der Inhalte des Mischbehälters (102) ein Messen einer Leitfähigkeit der Inhalte umfasst,
**dadurch gekennzeichnet, dass** das Messen der Leitfähigkeit der Inhalte umfasst
Pumpen einer ersten Menge der Inhalte durch einen Leitfähigkeitssensor (159, 159c, 159s) und erstes Messen einer Leitfähigkeit der ersten Menge der Inhalte mit dem Leitfähigkeitssensor (159, 159c, 159s);
als Reaktion auf eine Feststellung, dass ein Wert der gemessenen Leitfähigkeit der ersten Menge der Inhalte nicht größer als ein vordefinierter Wert ist, Pumpen einer zweiten Menge der Inhalte durch den Leitfähigkeitssensor (159, 159c, 159s) und Messen einer Leitfähigkeit der zweiten Menge der Inhalte mit dem Leitfähigkeitssensor (159, 159c, 159s);
als Reaktion auf eine Feststellung, dass sich die gemessene Leitfähigkeit der zweiten Menge der Inhalte um mehr als einen vordefinierten Bereich von der gemessenen Leitfähigkeit der ersten Menge der Inhalte unterscheidet, weiteres Mischen der Inhalte und anschließendes Pumpen einer dritten Menge der Inhalte durch den Leitfähigkeitssensor (159, 159c, 159s) sowie Messen einer Leitfähigkeit der dritten Menge der Inhalte mit dem Leitfähigkeitssensor (159, 159c, 159s);
als Reaktion auf eine Feststellung, dass ein Wert der gemessenen Leitfähigkeit der dritten Menge der Inhalte nicht größer ist als ein zweiter vordefinierter Wert, Pumpen einer vierten Menge der Inhalte durch den Leitfähigkeitssensor (159, 159c, 159s) und Messen einer Leitfähigkeit der vierten Menge der Inhalte mit dem Leitfähigkeitssensor (159, 159c, 159s); und
als Reaktion auf eine Feststellung, dass die gemessene Leitfähigkeit der vierten Menge der Inhalte um weniger als einen vordefinierten Bereich von der gemessenen Leitfähigkeit der dritten Menge der Inhalte abweicht, Ausgeben einer Messung, die auf der gemessenen Leitfähigkeit der dritten Menge der Inhalte und/oder der gemessenen Leitfähigkeit der vierten Menge der Inhalte basiert.

2. Verfahren nach Anspruch 1, ferner umfassend:
Verbinden einer ersten Quelle (310) des ersten konzentrierten Arzneimittels mit der Einwegkomponente (161) mittels eines Verbindungsstücks (124); und
Verbinden einer zweiten Quelle (316) des zweiten konzentrierten Arzneimittels mit der Einwegkomponente (161) mittels eines zweiten Verbindungsstücks (124).

3. Verfahren nach Anspruch 1, ferner umfassend:
Fördern einer variablen Menge des aufbereiteten Wassers in den Mischbehälter (102) nach dem ersten Mischen, wobei die variable Menge auf der Grundlage der bestimmten Konzentration der Inhalte bestimmt wird.

4. Verfahren nach Anspruch 3, ferner umfassend:
weiteres Bestimmen einer Konzentration der Inhalte zu einem Zeitpunkt nach Fördern der variablen Menge des aufbereiteten Wassers zu dem Mischbehälter (102) und vor Fördern der dritten Menge des zweiten konzentrierten Arzneimittels zu dem Mischbehälter (102).

5. Verfahren nach Anspruch 1, ferner umfassend:
Fördern einer zweiten variablen Menge des aufbereiteten Wassers zu dem Mischbehälter (102) nach dem zweiten Mischen, wobei die zweite variable Menge auf der Grundlage einer bestimmten Konzentration der Inhalte des Mischbehälters (102) nach dem zweiten Mischen bestimmt wird.

6. Verfahren nach Anspruch 1, wobei das Bereitstellen der Inhalte des Mischbehälters (102) an den Arzneimittelanwender (157) weniger als eine Stunde nach Beginn der Herstellung des Dialysats erfolgt.

7. Verfahren nach Anspruch 1, wobei das Fördernder dritten Menge des zweiten konzentrierten Arzneimittels zu dem Mischbehälter (102) umfasst, die dritte Menge des zweiten konzentrierten Arzneimittels zu dem Mischbehälter (102) zu fördern, als Reaktion darauf, dass das Bestimmen der Konzentration der Inhalte des Mischbehälters (102) anzeigt, dass kein grober Fehler bei einer Messung der Konzentration der Inhalte des Mischbehälters (102) vorliegt.

8. Verfahren nach Anspruch 1, wobei das erste konzentrierte Arzneimittel ein osmotisches Wirkstoffkonzentrat und das zweite konzentrierte Arzneimittel ein Elektrolytkonzentrat ist.

## Revendications

1. Procédé non thérapeutique permettant de produire un mini-lot personnalisé de dialysat à l'aide d'un système de dosage, le procédé comprenant :
fixation d'un composant jetable (161) au système de dosage ;
production de l'eau purifiée à l'aide d'un système de purification d'eau (133) ;
ajout d'une première quantité d'eau purifiée dans un récipient de mélange (102) préalablement fixé au composant jetable (161) ;
acheminement d'une deuxième quantité d'un premier médicament concentré vers le récipient de mélange (102) ;
un premier mélange des contenus du récipient de mélange (102) ;
détermination d'une concentration des contenus du récipient de mélange (102) ;
acheminement d'une troisième quantité d'un deuxième médicament concentré vers le récipient de mélange (102) ;
un deuxième mélange des contenus du récipient de mélange (102) ;
vérification de la concentration finale des contenus du récipient de mélange (102) ; et
approvisionnement des contenus du récipient de mélange (102) à un utilisateur du médicament (157), dans lequel
la détermination de la concentration des contenus du récipient de mélange (102) comprend la mesure de la conductivité des contenus,
**caractérisé en ce que** la mesure de la conductivité des contenus comprend
pompage d'une première quantité des contenus à travers un capteur de conductivité (159, 159c, 159s) et une première mesure, par le capteur de conductivité (159, 159c, 159s), de la conductivité de cette première quantité des contenus ;
en réponse à la détermination qu'une valeur de la conductivité mesurée de la première quantité des contenus n'est pas supérieure à une valeur prédéfinie, pompage d'une deuxième quantité des contenus à travers le capteur de conductivité (159, 159c, 159s) et mesure, par le capteur de conductivité (159, 159c, 159s), d'une conductivité de la deuxième quantité des contenus ;
en réponse à la détermination que la conductivité mesurée de la deuxième quantité des contenus diffère de la conductivité mesurée de la première quantité des contenus de plus d'une plage prédéfinie, mélanger davantage les contenus, puis pomper une troisième quantité des contenus à travers le capteur de conductivité (159, 159c, 159s) et mesure, à l'aide du capteur de conductivité (159, 159c, 159s), d'une conductivité d'une troisième quantité des contenus ;
en réponse à la détermination que la valeur de la conductivité mesurée de la troisième quantité du contenu n'est pas supérieure à une deuxième valeur prédéfinie, pompage une quatrième quantité des contenus à travers le capteur de conductivité (159, 159c, 159s) et mesure, à l'aide du capteur de conductivité (159, 159c, 159s), d'une conductivité d'une quatrième quantité des contenus; et
en réponse à la détermination que la conductivité mesurée de la quatrième quantité des contenus diffère de la conductivité mesurée de la troisième quantité des contenus de moins d'une plage prédéfinie, fournir une mesure basée sur l'une ou l'autre, ou les deux, de la conductivité mesurée de la troisième quantité des contenus et de la conductivité mesurée de la quatrième quantité des contenus.

2. Procédé selon la revendication 1, comprenant en outre :
raccordement d'une première source (310) du premier médicament concentré au composant jetable (161) à l'aide d'un connecteur (124) ; et
raccordement d'une deuxième source (316) du deuxième médicament concentré au composant jetable (161) à l'aide d'un deuxième connecteur (124).

3. Procédé selon la revendication 1, comprenant en outre :
acheminement d'une quantité variable d'eau purifiée vers le récipient de mélange (102) après le premier mélange, ladite quantité variable étant déterminée en fonction de la concentration déterminée des contenus.

4. Procédé selon la revendication 3, comprenant en outre :
détermination supplémentaire d'une concentration des contenus à un moment situé après l'acheminement de la quantité variable d'eau purifiée vers le récipient de mélange (102) et avant l'acheminement de la troisième quantité du deuxième médicament concentré vers le récipient de mélange (102).

5. Procédé selon la revendication 1, comprenant en outre :
acheminement d'une deuxième quantité variable d'eau purifiée vers le récipient de mélange (102) après le deuxième mélange, la deuxième quantité variable étant déterminée en fonction d'une concentration déterminée des contenus du récipient de mélange (102) après le deuxième mélange.

6. Procédé selon la revendication 1, dans lequel l'approvisionnement des contenus du récipient de mélange (102) à l'utilisateur du médicament (157) a lieu moins d'une heure après le début de la production du dialysat.

7. Procédé selon la revendication 1, dans lequel l'acheminement de la troisième quantité du deuxième médicament concentré vers le récipient de mélange (102) comprend l'acheminement de la troisième quantité du deuxième médicament concentré vers le récipient de mélange (102) en réponse à la détermination de la concentration des contenus du récipient de mélange (102) indiquant qu'il n'y a pas d'erreur grossière dans la mesure de la concentration des contenus du récipient de mélange (102).

8. Procédé selon la revendication 1, dans lequel le premier médicament concentré est un concentré d'agent osmotique et le deuxième médicament concentré est un concentré d'électrolytes.
